# EUROPEAN PATENT SPECIFICATION

(11) **EP 1 326 586 B1**
(45) Date of publication and mention of the grant of the patent: **11.03.2009**
(21) Application number: 01980449.1
(22) Date of filing: 28.09.2001
(51) Int. Cl.: A61K 9/20, A61K 31/415, A61K 31/135, A61P 37/08

(54) **NEW PHARMACEUTICAL COMPOSITIONS CONTAINING EPINASTINE AND PSEUDOEPHEDRINE**
PHARMAZEUTISCHE ZUSAMMENSETZUNGEN MIT EPINASTIN UND PSEUDOEPHEDRIN
NOUVELLES COMPOSITIONS PHARMACEUTIQUES CONTENANT DE L'EPINASTINE ET DE LA PSEUDOEPHEDRINE

(30) Priority: 06.10.2000 EP 00121828
(43) Date of publication of application: 16.07.2003
(73) Proprietor: Boehringer Ingelheim International GmbH, 55216 Ingelheim (DE)
(72) Inventor: ABELAIRA, Sara, 1686 Hurlingham, Buenos Aires (AR); BIANCHI, Daniel, 1425 Buenos Aires (AR); GEL, Francisco, (1824) Lanus Este, Buenos Aires (AR); DENKER, Victor, 1428 Buenos Aires (AR); FERNANDEZ, Mabel, 1605 Munro, Buenos Aires (AR); VIDAL, Marta, Cicconi de, 1754 San Justo, Buenos Aires (AR)
(86) International application number: PCT/EP2001/011229
(87) International publication number: WO 2002/028373

(56) References cited:
- EP-A- 0 903 151
- WO-A-01/51038
- WO-A-99/32125

## Description

### Background of the invention

The present invention relates to novel oral pharmaceutical compositions comprising as pharmaceutically active compounds a combination of an antihistaminic-effective amount of epinastine or a pharmaceutically acceptable salt thereof and of a decongestant-effective amount of pseudoephedrine or a pharmaceutically acceptable salt thereof and further comprising suitable pharmaceutically acceptable carriers or excipients.

WO 99/32125 discloses compositions comprising at least one leucotriene antagonist, epinastine and pseudoephedrine.

EP 0 903 151 A1 relates to combinations of non-sedating antihistamines, inter alia epinastine and alpha-adrenergic agonists.

WO 01/51038, puplished on 19.07.2001, discloses compositions comprising pseudoephedrine and as an H1 antagonist inter alia epinastine.

### Description of the invention

The present invention provides for novel oral pharmaceutical compositions comprising as pharmaceutically active compounds a combination of an antihistaminic-effective amount of epinastine or a pharmaceutically acceptable salt thereof and of a decongestant-effective amount of pseudoephedrine or a pharmaceutically accptable salt thereof and further comprising pharmaceutically acceptable carriers or excipients under the proviso that the composition does not contain a leukotriene antagonist.

As an additional active compound the compositions according to the invention may optionally contain one or several compounds selected from the group consiting of mucolitic and analgesic-antipyretic compounds and vitamines. Preferred mucolitic ingredients are selected from bromhexine and ambroxol. Preferred analgesic-antipyretic compounds are selected from paracetamol and obuprofen. Preferred vitamines are selected from vitamine B2, B6 and C.

The pharmaceutical compositions according to the invention are useful for the treatment of allergic rhinitis, allergic congestion of the Eustachian tubes and / or other diseases from allergic origin deserving the administration of antihistamine and decongestant drugs. Furthermore the compositions according to the invention are useful in the treatment of for instance common cold and in the symptomatic relief associated with cough, cold and flu symptoms. The use of the pharmaceutical compositions according to the invention for the treatment of allergic rhinitis, allergic congestion of the Eustachian tubes and / or other diseases from allergic origin deserving the administration of antihistamine and decongestant drugs is preferred.

In a preferred embodiment the pharmaceutical composition according to the invention contains as the active ingredients only an antihistaminic-effective amount of epinastine or a pharmaceutically acceptable salt thereof and a decongestant-effective amount of pseudoephedrine or a pharmaceutically accptable salt thereof

In a preferred embodiment the present invention relates to an oral pharmaceutical composition, preferably a bilayer tablet, providing for a sustained release of the decongestant effective amount of pseudoephedrine and an immediate release of an antihistaminic effective amount of epinastine.

Particularily preferred according to the invention is a bilayer tablet wherein a first layer A, providing for the sustained release of pseudoephedrine, comprises a decongestant effective amount of pseudoephedrine or a pharmaceutically acceptable salt thereof and wherein a second layer B, providing for the immediate release of epinastine, comprises an antihistaminic effective amount of epinastine or a pharmaceutically acceptable salt thereof. The bilayer tablet according to the invention may additionally contain a tablet coating C consisting of pharmaceutically acceptable excipients which mask the bitter taste of one of the active compound.

In a preferred embodiment of the invention layer A of the bilayer tablet according to the invention comprises a decongestant effective amount of pseudoephedrine or a pharmaceutically acceptable salt thereof in a matrix of a swellable hydrophilic polymer which provides a sustained release profile in a period of 3 to 24, preferably 6 to 18, most preferably about 12 hours.

According to the invention the term pharmaceutically acceptable salts stands for acid addition salts of the active compounds pseudoephedrine and epinastine. These acid addition salts can be formed with anorganic acids like hydrochloric acid, hydrobromic acid or sulfuric acid or with organic acids as for instance oxalic acid, fumaric acid or methansulfonic acid. Epinastine is preferably used as its hydrochloric acid addition salt. Pseudoephedrine is preferebly used as the hydrochloride or the sulfate. Within the present invention pseudoephedrine sulfate is most preferred.

The release of pseudoephedrine takes place over 3 to 24, preferably 6 to 18, most preferably about 12 hours. This bilayer tablet is designed to be preferably administered twice daily.

The concentration range of pseudoephedrine salt in the compositions according to the invention is between 5 and 240 mg/tablet, preferably 10 to 200 mg/tablet, more preferably 60 to 180 mg/tablet, preferably 80 to 140 mg/tablet, most preferably 120 mg/tablet. The concentration range of epinastine salt in the compositions according to the invention is between 2 and 20 mg/tablet, preferably 5 to 10 mg/tablet, more preferably 10 mg/tablet.

Each layer of the tablet is in contact with each other in a portion of their surface, but provides independent release profiles for both active substances mentioned before. The sustained release layer A consists of pseudoephedrine or a pharmaceutically acceptable salt thereof and a swellable hydrophilic polymer .

Typical swellable hydrophilic polymers include cellulosic ethers such as methylcellulose, hydroxypropylcellulose, hydroxypropylmethylcellulose, hydroxymethylcellulose, hydroxyethylcellulose, carboxymethylcellulose and carboxyethylcellulose or mixtures thereof. The use of hydroxypropylmethylcellulose (HPMC) is preferred. Particularly useful are the HPMC polymers HPMC USP2910 and USP2208 like for instance Methocel E5, E4M, E15M, K15M, and K100M supplied by the Dow Chemical Company. In the aformentioned abbreviations the designation "E" refers to USP2910 whereas "K" refers to USP2208. The number designation refers to the viscosity in a 2% aqueous solution (e.g. 5 designates a viscosity of 5 cps; 15M designates a viscosity of 15000 cps).

The excipients that could be optionally used in the sustained release layer A are insoluble polymers, soluble or insoluble fillers, antiadherents, coloring agents, lubricants and additional binders. Typical fillers are for example lactose, microcrystalline cellulose, dibasic calcium phosphate and cornstarch. Examples of antiadherents, which are used to prevent tablets from sticking to the tablet press, are colloidal silicon dioxide and talc. Magnesium stearate, talc and stearic acid are typical lubricants. Typical binders are povidone, and cornstarch.

The immediate release matrix layer B comprises epinastine within different combinations of excipients. The excipients that could be optionally used in the immediate release layer B are insoluble polymers, soluble or insoluble fillers, antiadherents, lubricants, coloring agents,disintegrants and additional binders. Typical fillers are for example lactose, microcrystalline cellulose, dibasic calcium phosphate and cornstarch. Examples of antiadherents, which are used to prevent tablets from sticking to the tablet press, are colloidal silicon dioxide and talc. Typical disintegrants are crospovidone, sodium starch glycolate and crosscarmellose sodium. Typical coloring agents are selected from FD&C red 40 HT Aluminum lake, 2-hydroxy-1,1'-azonaphthalene-3,6,4'-trisulfonic acid trisodium salt, erythrosine, iron oxides, 1-(4-sulpho-1-naphthylazo)-2-naphthol-6,8-disulphonic acid trisodium salt, 2',4',5',7'-tetrabromo-4,5,6,7-tetrachloro-fluorescein disodium salt, 2,4,5,7-Tetraiodo-3,6-dihydroxyxanthene-9-spiro-1'-(4',5',6',7'-tetrachloro-3'H-isobenzofuran-3'one dipotassium salt, trisodium 3-carboxy-5-hydroxy-1-p-sulphophenyl-4-p-sulfophenylazopyrazole, 6-hydroxy-5-((4-sulphonphenyl)azo-2-naphthalenesulphonic acid disodium salt and optionally aluminium lakes thereof. Magnesium stearate, talc and stearic acid are typical lubricants. Typical binders are povidone, and cornstarch.

Water and ethanol are examples of volatile components which can be used in the manufacture process of both layers to granulate powders. These volatile components are removed during processing and therefore do not appear in the finished product.

The tablet coating is optional since the presence of it does not modifies significantly the release rates of the active substances present in the core layers. The presence of the coating is preferred because it masks the bitter taste of one of the active substances and enhances the properties of dosage form. Because of that a lot different coatings with different polymers, and plasticizers and other excipients could be used with the condition of not modifying significantly the release profile of the active substances present in the core tablet. A typical coating comprises a polymer such as hydroxypropylmethylcellulose and a plasticizer such as polyethylene glycol.

Optional excipients could be added to the coating like antifoaming agents and opacifying. Example of an antifoaming agent is silicone. Examples of opacifying agents are Titanium dioxide, talc and aluminum lake dyes.

The invention will be further described by the following examples. These examples disclose certain preferred embodiments of the invention. The methods of manufacturing the compositions according to the invention like for instance granulation, tablet compression, tablet coating etc. are well known to the person skilled in the art. Those skilled in the art will appreciate that various changes, modifications and substitutions can be made therein without departing from the spirit of the invention. Accordingly, it is intended that the invention be not limited to the following explicitly disclosed examples.

### Example N°1 - Composition

### Core

| **A. First layer** | |
|---|---|
| **Layer pseudoephedrine** | **mg/tablet** |
| Pseudoephedrine sulfate | 120.00 |
| Methocel K 15 M PRCR * | 198.00 |
| Lactose Monohydrate | 105.10 |
| Microcrystalline cellulose | 106.00 |
| Colloidal silicon dioxide | 1.65 |
| Magnesium Stearate | 2.75 |
| Povidone | 16.50 |
| Total first layer | **550.00** |
| | |

| **B. Second layer** | |
|---|---|
| **Layer Epinastine** | **mg / tablet** |
| Epinastine HCl | 10.00 |
| FD&C red 40 HT Aluminum lake (allura red | 0.38 |
| AC) | |
| Microcrystalline cellulose | 70.00 |
| Lactose Monohydrate | 154.62 |
| Povidone | 12.50 |
| Magnesium Stearate | 2.50 |
| Total second layer | **250.00** |
| | |
| **Total core Coating** | **800.00** |

| **C.** | |
|---|---|
| **Film Coating** | **mg/ tablet** |
| Methocel E5 | 15.00 |
| Polyethylene Glycol 6000 | 1.97 |
| Silicone antifoam S184 | 0.03 |
| Total film coating | **17.00** |
| | |
| **Total Film coated tablet** | **817.00** |

| | |
|---|---|
| * PR means Premium grade and CR means Controlled Released grade. | |

### Method of Manufacture

### A. First layer:

A1. Dissolve povidone in a hydroalcoholic mixture;
A2. Blend pseudoephedrine sulfate, a portion of the microcrystalline cellulose, lactose and Methocel K15M for 5-30 minutes in a suitable mixer.
A3. Use alcoholic or hydroalcoholic solution prepared previously in step A1. to granulate the powder mix.
A4. Dry and mill the pseudoephedrine sulfate granulation from step A3, using suitable size screen.
A5. Blend the screened pseudoephedrine sulfate granulation with a portion of the microcrystalline cellulose and colloidal silicon dioxide for 3-15 minutes.
A6. Add magnesium stearate and blend for 3-15 minutes.

### B Second layer:

B1. Pass through a suitable screen Epinastine HCL, Allura red AC (FD & C red 40 HT) aluminum lake and microcrystalline cellulose. Blend for 5-30 minutes in a suitable mixer.
B2. Add lactose and povidone. Blend for 60 minutes 15-120 minutes in a suitable mixer.
B3. Add magnesium stearate. Blend for 3-20 minutes in a suitable mixer.

### C. Compression:

Compress A and B into a suitable bilayer tableting machine in suitable size tablets.

### D. Coating

D1. Dissolve Methocel E5 and Polyethylene Glycol in suitable amount of water. D2. Dissolve silicone antifoam in suitable amount of isopropilic alcohol.
D3. Add 2. to 1. and mix.
D4. Coat tablets with the Methocel E5 /Polyethylene glycol solution from step D3. in a suitable coater.

### Example N° 2 - Composition

### Core

| **A. First layer** | |
|---|---|
| **Layer pseudoephedrine** | **Mg/tablet** |
| Pseudoephedrine sulfate | 120.00 |
| Methocel K 15 M PRCR * | 198.00 |
| Lactose Monohydrate | 126.50 |
| Microcrystalline cellulose | 100.00 |
| Colloidal silicon dioxide | 2.75 |
| Magnesium Stearate | 2.75 |
| Total first layer | **550.00** |
| | |

| **B. Second layer** | |
|---|---|
| **Layer Epinastine** | **Mg / tablet** |
| Epinastine HCl | 10.00 |
| Lactose Monohydrate | 168.40 |
| Microcrystalline cellulose | 70.00 |
| Punceau 4R red aluminum lake | 0.38 |
| Magnesium Stearate | 1.25 |
| Total second layer | **250.00** |
| | |
| **Total core** | **800.00** |

| **C. Coating** | |
|---|---|
| **Film Coating** | **mg/ tablet** |
| Methocel E5 | 4.42 |
| Polyethylene Glycol 6000 | 2.72 |
| Talc | 8.76 |
| Titanium dioxide | 1.10 |
| Total film coating | **17.00** |
| | |
| **Total Film coated tablet** | **817.00** |

| | |
|---|---|
| * PR means Premium grade and CR means Controlled Released grade. | |

### Method of Manufacture

### A. First laver:

A1. Blend pseudoephedrine sulfate, microcrystalline cellulose, lactose, colloidal silicon dioxide and HPMC K15M for 5-30 minutes in a suitable mixer.
A2. Add magnesium stearate and blend for 3-15 minutes.

### B. Second layer:

B1. Pass through a suitable screen Epinastine HCl, and microcrystalline cellulose. Blend for 5-30 minutes in a suitable mixer.
B2. Add lactose. Blend for 60 minutes 15-120 minutes in a suitable mixer.
B3. Add magnesium stearate. Blend for 3-20 minutes in a suitable mixer.

### C. Compression:

Compress A and B into a suitable bilayer tableting machine in suitable size tablets.

### D. Coating

D1. Dissolve Methocel E5 and Polyethylene Glycol in suitable amount of water.
D2. Add Titanium Dioxide and Talc in suitable amount of water and mix
D3. Add 2. to 1. And mix.
D4. Coat tablets with the Methocel E5 /Polyethylene glycol solution from step D3. in a suitable coater.

### Example N° 3

### Core

| **A. First layer** | |
|---|---|
| **Layer pseudoephedrine** | **Mg/tablet** |
| Pseudoephedrine sulfate | 120.00 |
| Methocel K4M PRCR | 247.50 |
| Lactose Monohydrate | 166.00 |
| Talc | 11.00 |
| Magnesium Stearate | 5.50 |
| **Total first layer** | **550.00** |

| | |
|---|---|
| * PR means Premium grade and CR means Controlled Released grade. | |

Second layer and coating are identical to example 2; the manufacture method was conducted analogously to the method outlined in example 2;

### Example N° 4

### Core

| **A. First layer** | |
|---|---|
| **Layer pseudoephedrine** | **Mg/tablet** |
| Pseudoephedrine sulfate | 120.00 |
| Methocel K15 M PRCR | 198.00 |
| Lactose Monohydrate | 99.50 |
| Microcrystalline cellulose | 99.50 |
| Colloidal silicon dioxide | 2.75 |
| Povidone | 27.50 |
| Magnesium stearate | 2.75 |
| **Total** | **550.00** |

| | |
|---|---|
| * PR means Premium grade and CR means Controlled Released grade. | |

Second layer and coating are identical to example 1; the manufacture method was conducted analogously to the method outlined in example 1;

### Example N° 5

### Core

| **A. First layer** | |
|---|---|
| **Layer pseudoephedrine** | **Mg/tablet** |
| Pseudoephedrine sulfate | 120.00 |
| Methocel K15M CR | 330.00 |
| Lactose | 83.50 |
| Talc | 11.00 |
| Magnesium Stearate | 5.50 |
| **Total** | **550.00** |

| | |
|---|---|
| * CR means Controlled Released grade. | |

Second layer and coating are identical to example 1; the manufacture method was conducted analogously to the method outlined in example 1;

### Example N° 6

### Core

| **A. First layer** | |
|---|---|
| **Layer pseudoephedrine** | **Mg/tablet** |
| Pseudoephedrine sulfate | 120.00 |
| Methocel K15M CR | 275.00 |
| Microcrystalline Cellulose | 138.50 |
| Talc | 11.00 |
| Magnesium Stearate | 5.50 |
| Ethanol | sq. |
| **Total** | **550.00** |

| | |
|---|---|
| * CR means Controlled Released grade. | |

Second layer and coating are identical to example 1; the manufacture method was conducted analogously to the method outlined in example 1;

### Example N° 7

### Core

| **A. First layer** | |
|---|---|
| **Layer pseudoephedrine** | **Mg/tablet** |
| Pseudoephedrine sulfate | 120.00 |
| Methocel K15M CR | 215.00 |
| Dibasic Calcium phosphate | 108.50 |
| Ethylcelullose | 40.00 |
| Talc | 11.00 |
| Magnesium Stearate | 5.50 |
| Ethanol | s.q. |
| **Total** | **500.00** |

| | |
|---|---|
| * CR means Controlled Released grade. | |

Second layer and coating are identical to example 1; the manufacture method was conducted analogously to the method outlined in example 1;

### Example N° 8

### Core

| | |
|---|---|
| **A. First layer** | |

| **Layer pseudoephedrine** | **Mg/tablet** |
|---|---|
| Pseudoephedrine sulfate | 120.00 |
| Methocel K15M CR | 137.50 |
| Methocel K100M CR | 137.50 |
| Lactose | 138.50 |
| Talc | 11.00 |
| Magnesium Stearate | 5.50 |
| Ethanol | s.q. |
| **Total** | **550.00** |

| | |
|---|---|
| * CR means Controlled Released grade. | |

Second layer and coating are identical to example 1; the manufacture method was conducted analogously to the method outlined in example 1;

### Example N° 9

### Core

| **A. First layer** | |
|---|---|
| **Layer pseudoephedrine** | **Mg/tablet** |
| Pseudoephedrine sulfate | 120.00 |
| Methocel K100M CR | 275.00 |
| Lactose | 138.50 |
| Talc | 11.00 |
| Magnesium Stearate | 5.50 |
| Ethanol | s.q. |
| **Total** | **550.00** |

| | |
|---|---|
| * CR means Controlled Released grade. | |

Second layer and coating are identical to example 1; the manufacture method was conducted analogously to the method outlined in example 1;

### Example N° 10

### Core

| **A. First layer** | |
|---|---|
| **Layer pseudoephedrine** | **Mg/tablet** |
| Pseudoephedrine sulfate | 120.00 |
| Methocel K15M CR | 206.20 |
| Methocel K100M CR | 68.80 |
| Lactose | 138.50 |
| Talc | 11.00 |
| Magnesium Stearate | 5.50 |
| Ethanol | s.q. |
| **Total** | **550.00** |

| | |
|---|---|
| * CR means Controlled Released grade. | |

Second layer and coating are identical to example 1; the manufacture method was conducted analogously to the method outlined in example 1;

### Example N° 11

### Core

| **A. First layer** | |
|---|---|
| **Layer pseudoephedrine** | **Mg/tablet** |
| Pseudoephedrine sulfate | 120.00 |
| Methocel K15M CR | 235.00 |
| Dibasic Calcium phosphate | 108.50 |
| Ethylcellulose | 20.00 |
| Talc | 11.00 |
| Magnesium Stearate | 5.50 |
| Ethanol | s.q. |
| **Total** | **500.00** |

| | |
|---|---|
| * CR means Controlled Released grade. | |

Second layer and coating are identical to example 1; the manufacture method was conducted analogously to the method outlined in example 1;

### Example N° 12

### Core

| **A. First layer** | |
|---|---|
| **Layer pseudoephedrine** | **Mg/tablet** |
| Pseudoephedrine sulfate | 120.00 |
| Methocel K15M CR | 255.00 |
| Lactose | 40.00 |
| Microcrystalline Cellulose | 68.50 |
| Talc | 11.00 |
| Magnesium Stearate | 5.50 |
| Ethanol | s.q. |
| **Total** | **500.00** |

| | |
|---|---|
| * CR means Controlled Released grade. | |

Second layer and coating are identical to example 1; the manufacture method was conducted analogously to the method outlined in example 1;

### Example N° 13

### Core

| **A. First layer** | |
|---|---|
| **Layer pseudoephedrine** | **Mg/tablet** |
| Pseudoephedrine sulfate | 120.00 |
| Methocel K15M CR | 255.00 |
| Dibasic calcium phosphate | 108.50 |
| Talc | 11.00 |
| Magnesium Stearate | 5.50 |
| Ethanol | s.q. |
| **Total** | **500.00** |

| | |
|---|---|
| * CR means Controlled Released grade. | |

Second layer and coating are identical to example 1; the manufacture method was conducted analogously to the method outlined in example 1;

## Claims

1. Oral pharmaceutical compositions comprising as pharmaceutically active compounds a combination of an antihistaminic-effective amount of epinastine or a pharmaceutically acceptable salt thereof and of a decongestant-effective amount of pseudoephedrine or a pharmaceutically acceptable salt thereof and further comprising pharmaceutically acceptable carriers or excipients under the proviso that the composition does not contain a leukotriene antagonist.

2. Oral pharmaceutical composition according to claim 1, **characterized in that** it contains as the active ingredients only an antihistaminic-effective amount of epinastine or a pharmaceutically acceptable salt thereof and a decongestant-effective amount of pseudoephedrine or a pharmaceutically accptable salt thereof.

3. Oral pharmaceutical composition according to claim 1 or 2, providing for a sustained release of the decongestant effective amount of pseudoephedrine and an immediate release of an antihistaminic effective amount of epinastine.

4. Oral pharmaceutical composition according to claims 1, 2 or 3, **characterized in that** it represents a bilayer tablet.

5. Bilayer tablet according to claim 4, wherein a first layer A, providing for the sustained release of pseudoephedrine, comprises a decongestant effective amount of pseudoephedrine or a pharmaceutically acceptable salt thereof and wherein a second layer B, providing for the immediate release of epinastine, comprises an antihistaminic effective amount of epinastine or a pharmaceutically acceptable salt thereof.

6. Bilayer tablet according to claim 5, **characterized in that** it additionally contains a tablet coating C consisting of pharmaceutically acceptable excipients.

7. Bilayer tablet according to claim 4, 5 or 6, **characterized in that** layer A comprises a decongestant effective amount of pseudoephedrine or a pharmaceutically acceptable salt thereof in a matrix of a swellable hydrophilic polymer.

8. Bilayer tablet according to claim 4, 5, 6 or 7, **characterized in that** the concentration range of pseudoephedrine salt is 5 and 240 mg/tablet and the concentration range of epinastine salt in the compositions according to the invention is between 2 and 20 mg/tablet.

9. Bilayer tablet according to one of claims 4 to 8, **characterized in that** layer A comprises 120 mg pseudoephedrine sulfate and layer B comprises 10 mg epinastine-HCl.

10. A pharmaceutical composition according to one of claims 1 to 9 for the treatment of allergic rhinitis, allergic congestion of the Eustachian tubes and / or other diseases from allergic origin deserving the administration of antihistamine and decongestant drugs, in the treatment of for instance common cold and in the symptomatic relief associated with cough, cold and flu symptoms.

11. A pharmaceutical composition according to claim 10 for the treatment of allergic rhinitis, allergic congestion of the Eustachian tubes and / or other diseases from allergic origin deserving the administration of antihistamine and decongestant drugs, preferably of allergic rhinitis.

## Patentansprüche

1. Orale pharmazeutische Zusammensetzungen, umfassend als pharmazeutisch aktive Verbindungen eine Kombination einer antihistaminisch wirksamen Menge von Epinastin oder eines pharmazeutisch akzeptablen Salzes hiervon und einer abschwellend wirkenden Menge von Pseudoephedrin oder eines pharmazeutisch akzeptablen Salzes hiervon, und weiterhin umfassend pharmazeutisch akzeptable Träger oder Hilfsstoffe, mit der Maßgabe, dass die Zusammensetzung keinen Leukotrien-Antagonisten enthält.

2. Orale pharmazeutische Zusammensetzung nach Anspruch 1, **dadurch gekennzeichnet, dass** diese als aktive Bestandteile nur eine antihistaminisch wirksamen Menge von Epinastin oder eines pharmazeutisch akzeptablen Salzes hiervon und einer abschwellend wirkenden Menge von Pseudoephedrin oder eines pharmazeutisch akzeptablen Salzes hiervon enthält.

3. Orale pharmazeutische Zusammensetzung nach Anspruch 1 oder 2, die eine verzögerte Freisetzung der abschwellend wirkenden Menge von Pseudoephedrin und eine sofortige Freisetzung der antihistaminisch wirksamen Menge von Epinastin bereitstellt.

4. Orale pharmazeutische Zusammensetzung nach Anspruch 1, 2 oder 3, **dadurch gekennzeichnet, dass** diese eine Zweischicht-Tablette darstellt.

5. Zweischicht-Tablette nach Anspruch 4, wobei die erste Schicht A, welche die verzögerte Freisetzung des Pseudoephedrins bereitstellt, eine abschwellend wirkende Menge von Pseudoephedrin oder eines pharmazeutisch akzeptablen Salzes hiervon aufweist, und wobei die zweite Schicht B, welche die sofortige Freisetzung des Epinastins bereitstellt, eine antihistaminisch wirkende Menge von Epinastin oder eines pharmazeutisch akzeptablen Salzes hiervon aufweist.

6. Zweischicht-Tablette nach Anspruch 5, **dadurch gekennzeichnet, dass** diese zusätzlich eine Tablettenbeschichtung C enthält, die aus pharmazeutisch akzeptablen Hilfsstoffen besteht.

7. Zweischicht-Tablette nach Anspruch 4, 5 oder 6, **dadurch gekennzeichnet, dass** Schicht A eine abschwellend wirkende Menge von Pseudoephedrin oder eines pharmazeutisch akzeptablen Salzes hiervon in einer Matrix eines quellbaren hydrophilen Polymers aufweist.

8. Zweischicht-Tablette nach Anspruch 4, 5, 6 oder 7, **dadurch gekennzeichnet, dass** der Konzentrationsbereich des Pseudoephedrinsalzes 5 bis 240 mg/Tablette beträgt und der Konzentrationsbereich des Epinastinsalzes in der erfindungsgemäßen Zusammensetzung zwischen 2 und 20 mg/Tablette liegt.

9. Zweischicht-Tablette nach einem der Ansprüche 4 bis 8, **dadurch gekennzeichnet, dass** Schicht A 120 mg Pseudoephedrinsulfat umfasst und Schicht B 10 mg Epinastin-HCl umfasst.

10. Pharmazeutische Zusammensetzung nach einem der Ansprüche 1 bis 9 zur Behandlung von allergischer Rhinitis, allergischer Kongestion der eustachischen Röhren und/oder anderen Erkrankungen allergischen Ursprungs, die der Verabreichung von Antihistamin und abschwellenden Arzneimitteln bedürfen, bei der Behandlung von beispielsweise Erkältungen bzw. grippalen Infekten und bei der symptomatischen Linderung in Zusammenhang mit Husten-, Erkältungs- und Grippesymptomen.

11. Pharmazeutische Zusammensetzung nach Anspruch 10 zur Behandlung von allergischer Rhinitis, allergischer Kongestion der eustachischen Röhren und/oder anderen Erkrankungen allergischen Ursprungs, die der Verabreichung von Antihistamin und abschwellenden Arzneimitteln bedürfen, bevorzugt von allergischer Rhinitis.

## Revendications

1. Compositions pharmaceutiques orales comprenant comme composés pharmaceutiquement actifs une combinaison d'une quantité efficace du point de vue antihistaminique d'épinastine ou d'un sel pharmaceutiquement acceptable de celle-ci et d'une quantité efficace du point de vue décongestionnant de pseudoéphédrine ou d'un sel pharmaceutiquement acceptable de celle-ci et comprenant en outre des vecteurs ou excipients pharmaceutiquement acceptables à condition que la composition ne contienne pas d'antagoniste des leucotriènes.

2. Composition pharmaceutique orale selon la revendication 1 **caractérisée en ce qu'**elle contient comme ingrédients actifs seulement une quantité efficace du point de vue antihistaminique d'épinastine ou d'un sel pharmaceutiquement acceptable de celle-ci et une quantité efficace du point de vue décongestionnant de pseudoéphédrine ou d'un sel pharmaceutiquement acceptable de celle-ci.

3. Composition pharmaceutique orale selon la revendication 1 ou 2 assurant une libération prolongée de la quantité efficace du point de vue décongestionnant de pseudoéphédrine et une libération immédiate d'une quantité efficace du point de vue antihistaminique d'épinastine.

4. Composition pharmaceutique orale selon la revendication 1, 2 ou 3 **caractérisée en ce qu'**elle représente un comprimé bicouche.

5. Comprimé bicouche selon la revendication 4 où une première couche A, assurant la libération prolongée de pseudoéphédrine, comprend une quantité efficace du point de vue décongestionnant de pseudoéphédrine ou d'un sel pharmaceutiquement acceptable de celle-ci et où une seconde couche B, assurant la libération immédiate d'épinastine, comprend une quantité efficace du point de vue antihistaminique d'épinastine ou d'un sel pharmaceutiquement acceptable de celle-ci.

6. Comprimé bicouche selon la revendication 5 **caractérisé en ce qu'**il contient en outre un enrobage de comprimé C consistant en excipients pharmaceutiquement acceptables.

7. Comprimé bicouche selon la revendication 4, 5 ou 6 **caractérisé en ce que** la couche A comprend une quantité efficace du point de vue décongestionnant de pseudoéphédrine ou d'un sel pharmaceutiquement acceptable de celle-ci dans une matrice d'un polymère hydrophile capable de gonfler.

8. Comprimé bicouche selon la revendication 4, 5, 6 ou 7 **caractérisé en ce que** la plage de concentration de sel de pseudoéphédrine est 5 à 240 mg/comprimé et la plage de concentration de sel d'épinastine dans les compositions selon l'invention est entre 2 et 20 mg/comprimé.

9. Comprimé bicouche selon l'une des revendications 4 à 8 **caractérisé en ce que** la couche A comprend 120 mg de sulfate de pseudoéphédrine et la couche B comprend 10 mg d'épinastine-HCl.

10. Composition pharmaceutique selon l'une des revendications 1 à 9 pour le traitement de la rhinite allergique, de la congestion allergique des trompes d'Eustache et/ou d'autres maladies d'origine allergique justifiant l'administration de médicaments antihistaminiques et décongestionnants, dans le traitement par exemple du rhume et dans le soulagement symptomatique associé avec des symptômes de toux, de rhume et de grippe.

11. Composition pharmaceutique selon la revendication 10 pour le traitement de la rhinite allergique, de la congestion allergique des trompes d'Eustache et/ou d'autres maladies d'origine allergique justifiant l'administration de médicaments antihistaminiques et décongestionnants, de préférence la rhinite allergique.
